# EUROPÄISCHE PATENTANMELDUNG

(11) **EP 2 478 829 A1**
(43) Veröffentlichungstag der Anmeldung: **25.07.2012**
(21) Anmeldenummer: 11000481.9
(22) Anmeldetag: 21.01.2011
(51) Int. Cl.: A61B 1/31, A61B 1/01

(54) **Vorrichtung zur Einführung eines Instrumentes in einen Hohlraum**

(71) Anmelder: B. Braun Melsungen AG, 34212 Melsungen (DE)
(72) Erfinder: Böttger, Hans-Christian, Dr., 58313 Herdecke (DE); Grosser, Lars, 34212 Melsungen (DE); Fuchs, Jürgen, 34308 Bad Emstal (DE)

(57) **Zusammenfassung**

Beschrieben wird eine Vorrichtung zur Einführung eines daran befestigbaren oder integrierbaren länglichen, zylindrischen Instrumentes, insbesondere eines flexiblen Endoskops, in einen Hohlraum mit einem proximalen Griffteil, einem distalen Kopfteil und einem mehrlagigen wenigstens aus einem Schlauch bestehendem Schlauchgebilde.

## Beschreibung

Die Erfindung betrifft eine Vorrichtung zur Einführung eines daran befestigbaren oder integrierbaren länglichen, zylindrischen Instrumentes, insbesondere eines flexiblen Endoskops, in einen Hohlraum mit einem proximalen Griffteil, einem distalen Kopfteil und einem mehrlagigen wenigstens aus einem Schlauch bestehendem Schlauchgebilde.

Bei der endoskopischen Untersuchung, z.B. des Dickdarmes, wird entgegen der natürlichen Entleerungsrichtung des Darmes ein medizinisches Instrument, ein sogenanntes Koloskop, oder allgemeiner ein Endoskop, welches ein flexibles Schlauchgebilde mit steuerbarer Spitze aufweist, bis zur Dünndarmeinmündung und dort zum Teil hinein vorgeschoben. Dabei wird aufgrund der Reibung zwischen der Darmwand und dem Endoskop der Darm zurückgestreift, was Schmerzen während der Untersuchung erzeugen kann. Im Bereich der Kurvaturen, aber auch an anderen nicht geraden Darmabschnitten wird der Darm so gelängt und abgekrümmt, dass das weiterführende Lumen nicht ohne weiteres erkennbar ist. Der Darm wird im Bereich der Krümmungen vor dem Instrument hergeschoben, was die Sicht verlegt und wiederum Schmerzen verursacht. Beim weiteren Vorarbeiten des Endoskops wird das Endoskop von rektal weiter vorgeschoben. Die Schubrichtung weicht dabei in der Regel von der Schubrichtung der Gerätespitze meist erheblich ab. Befindet sich die Gerätespitze im Bereich des sogenannten Colon Ascendenz, liegt die Schubrichtung, mit der das Instrument vorgeschoben wird, sogar genau entgegengesetzt der Vorwärtsbewegung des Instrumentenkopfes. Dies hat für den untersuchten Patienten schmerzhafte erhebliche Dehnungen im Bereich der Kurvaturen zur Folge. Bedingt durch das Vorstehende muss der Untersucher durch zeitaufwendige Manöver versuchen, das Instrument zu begradigen, indem er es unter Umständen unter Terrainverlusten zurückzieht, um den Darm gewissermaßen aufzufädeln und um dann das Instrument erneut vorzuschieben. Häufig muss zusätzlich mit einem Röntgenbildwandler die aktuelle Lage des Instrumentes erst herausgefunden werden, um dann entsprechende Korrekturen vorzunehmen. Diese Manipulationen führen zu Schmerzen, langen Untersuchungszeiten, ggf. zum Arzneimitteleinsatz zur Analgesie, Sedierung oder gar zur Narkose, zu Strahlenbelastung und insgesamt zu einem erhöhten Eingriffsrisiko. Um eine solche Untersuchungstechnik einigermaßen beherrschen zu können, ist eine lange Trainingszeit notwendig. In dieser Trainingsphase werden, gemessen am geübten Untersucher, unnötige Komplikationen verursacht. Ähnliche Probleme treten auch bei Einsatz derartiger Instrumente in anderen Bereichen auf, bei denen ein Endoskop oder ein vergleichbares Instrument in einen länglichen, Krümmungen aufweisenden Hohlraum eingeführt wird.

### Stand der Technik

Zur Vermeidung der vorgeschilderten Probleme ist aus DE 28 23 025 C2 eine gattungsgemäße Vorrichtung bekannt geworden. Diese Vorrichtung zum Einführen eines Endoskops zur Darmdiagnostik besteht aus einem rohrförmigen Gehäuse, an dessen distaler Öffnung ein dünnwandiger Schlauch angebracht ist. Dieser Schlauch ist in das Innere des Gehäuses gestülpt und ist an seinem anderen Ende mit dem distalen Ende eines Endoskops verbunden. Durch Beaufschlagung des Gehäuses mit einem Druckmedium stülpt sich der Schlauch aus dem Gehäuse in die Körperöffnung, wobei das Endoskop nachgezogen wird. Der Schlauch kleidet mit fortschreitender Ausstülpung die Körperhöhle aus, so dass das Endoskop beim Eindringen in die Körperhöhle mit deren Wandung nicht in Berührung kommt. Dieses an sich für die Wandung der Körperhöhle sehr schonende Verfahren hat jedoch den wesentlichen Nachteil, dass der Anwender durch das Endoskop erst dann die Wandung der Körperhöhle inspizieren kann, wenn der Schlauch völlig ausgestülpt ist.

Zur Vermeidung der Nachteile ist aus DE 33 29 176 C1 ein längliches medizinisches Gerät zum Untersuchen oder Behandeln von Körperhöhlen mit einer Vorrichtung nach dem Prinzip des sich ausstülpenden Schlauchgebildes bekannt geworden, bei dem die Vorrichtung aus einem beidseitig offenen Rohr mit seitlichem Anschlussstutzen und einem durch das Rohr laufenden, flexiblen, ausstülpbaren Schlauchgebilde besteht, dessen beide Enden mit jeweils einem Ende des Rohres verbunden sind, wobei das medizinische Gerät innerhalb des Schlauchgebildes durch das Rohr läuft, wobei das Schlauchgebilde im Bereich des distalen Endes des medizinischen Gerätes in mehreren Doppellagen aufgefaltet ist. Dieses Gerät soll konstruktiv und in der Anwendung einfacher sein als das Vorbeschriebene. Bei diesem Gerät wird von der Tatsache Gebrauch gemacht, dass es nicht während des gesamten Vorschubes notwendig ist, den sich ausstülpenden Schlauch zu unterstützen, das medizinische Gerät wird vielmehr bei geradlinigen Passagen der Körperhöhlung auf konventionelle Weise vorgeschoben und nur bei problematischen Passagen mit Hilfe der Vorrichtung vorwärts bewegt. Erkennbar ist dieses bekannte Gerät deshalb nicht besonders leicht zu handhaben, da es nur zeitweise eingesetzt wird, während über einen Teilbereich der Vorschubbewegung das medizinische Instrument auf herkömmliche Weise durch Vorschieben bewegt wird.

Aus WO 02/19899 A2 ist eine Vorrichtung bekannt, welche zwei ineinander angeordnete Schläuche aufweist, die das Koloskop umgeben. Die Vorschubbewegung ist sehr kompliziert und erfolgt diskontinuierlich. Beide Schläuche können in Anpassung an den Verlauf des Hohlraumes, in den die Vorrichtung einzuführen ist, vorgekrümmt sein. Dies kann beim Ausstülpvorgang jedoch dazu führen, dass die Präformation des inneren Schlauches von der des äußeren Schlauches abweicht, was die Handhabung erschwert. Außerdem kann sich die präformierte Stelle während des langen Weges bis zum ausgestülpten endgültigen Ort verdrehen und dann in eine andere, nicht gewünschte Richtung zeigen. Diese z.B. als Wegwerfartikel konzipierte Vorrichtung ist somit nachteilig.

Zur Vermeidung der vorgenannten Probleme ist aus DE 103 53 957 A1 eine gattungsgemäße Vorrichtung bekannt geworden. Bei dieser Vorrichtung wird ein Folienschlauch von der Spitze des Endoskops ausgehend sukzessive beim Voranschieben des Endoskops über dasselbe ausgezogen. Hierdurch wird zwar die Reibung zwischen Außenwand des Instrumentes und Darminnenwandung aufgehoben, die Reibung zwischen der Folie und Endoskopaußenseite bleibt jedoch erhalten. Ferner kann das Endoskop beim Einführen unter Umständen mit einer Flüssigkeit, beispielsweise durch feuchte Handschuhe verursacht, benetzt werden, was zu Kapillaradhäsion mit stark erhöhtem Gleitwiderstand zwischen der Folie und der Außenseite des Endoskops führt.

### Definitionen

Unter dem Begriff "distal" im Sinne der vorliegenden Erfindung wird insbesondere eine Lage- und Richtungsbezeichnung verstanden, die ausgehend vom Anwender von der Körpermitte weg gerichtet bedeutet.

Unter dem Begriff "proximal" im Sinne der vorliegenden Erfindung wird insbesondere eine Lage- und Richtungsbezeichnung verstanden, die ausgehend vom Anwender zum Körper hin gelegen bedeutet.

Unter dem Begriff "distales Ende" im Sinne der vorliegenden Erfindung wird insbesondere sowohl ein bestimmter Punkt, als auch ein Bereich verstanden, der distal gelegen ist.

Unter dem Begriff "proximales Ende" im Sinne der vorliegenden Erfindung wird insbesondere sowohl ein bestimmter Punkt, als auch ein Bereich verstanden, der proximal gelegen ist.

Unter dem Term "komplementäres Wirkelement" im Sinne der vorliegenden Erfindung wird insbesondere ein Element, das so ausgestaltet ist, dass es mit einer "komplementären", d.h. zugehörigen, Befestigungsvorrichtung in Wirkeingriff bringbar ist, d.h. an die "komplementäre" Befestigungsvorrichtung reversibel fixierbar ist, verstanden.

Unter dem Term "Gewinde" im Sinne der vorliegenden Erfindung wird insbesondere eine profilierte Einkerbung verstanden, die fortlaufend wendelartig um eine zylinderförmige Wandung - innen oder außen - in einer Schraubenlinie verläuft. Die Kerbe wird als Gewindegang bezeichnet. Teile mit Außengewinde, beispielsweise Schrauben, und Teile mit Innengewinde, beispielsweise Muttern, müssen zueinander passen.

Unter dem Term "Bajonettverschluss" im Sinne der vorliegenden Erfindung wird insbesondere eine schnell herstell- und lösbare mechanische Verbindung zweier zylindrischer Teile in ihrer Längsachse verstanden. Die Teile werden durch Ineinanderstecken und entgegengesetztes Drehen verbunden und so auch wieder getrennt. Der Teil, der über den anderen geschoben wird, besitzt einen Längsschlitz, an dessen Ende sich rechtwinklig ein kurzer Querschlitz ansetzt. Der andere Teil besitzt dagegen einen Knopf, der in den Querschlitz eingeführt wird und dann die feste Verbindung bewirkt. Die Verbindung erfolgt über eine Steck-Dreh-Bewegung: Die beiden zu verbindenden Teile werden ineinandergesetzt; annähernd senkrecht zur Steckrichtung sind in beiden Teilen an der Verbindungsstelle längliche Erhebungen angebracht. Diese laufen jedoch nicht rundum, sondern sind unterbrochen. Da die Erhebungen leicht schräg in der Ebene senkrecht zur Steckrichtung liegen, werden durch eine Drehbewegung beide Teile gegeneinandergepresst. Der Bajonettverschluss arbeitet also wie ein Gewinde.

Unter dem Term "Adhäsionsmittel" im Sinne der vorliegenden Erfindung wird insbesondere ein Stoff verstanden, welcher auf einem Untergrund aufgrund von Adhäsions- oder Anhangskraft anhaftet. Adhäsion umfasst die Haftkräfte an den Kontaktflächen zweier unterschiedlicher oder gleicher Stoffe durch Molekularkräfte. Die Stoffe können sich in festem oder in flüssigem Zustand befinden. Im Bereich der Klebstoffe versteht man unter Adhäsion die Haftung von Klebschichten an den Fügeteiloberflächen.

Unter dem Term "Klettband" im Sinne der vorliegenden Erfindung wird insbesondere eine Befestigungsvorrichtung verstanden, welche eine Vielzahl winziger elastischer Haken aufweist, die sich in die kleinen weichen Schlaufen des hierzu komplementären Wirkelementes, welches als "Flauschband" bezeichnet wird, einhaken können.

Unter dem Term "Druckmanschette" im Sinne der vorliegenden Erfindung wird insbesondere ein ringförmiger elastischer Hohlraum verstanden, der durch Druckbeaufschlagung an Volumen zunimmt und dadurch eine Klemmung bewirkt. Die Druckbeaufschlagung erfolgt dabei mittels Aggregatszustandsänderung, d. h. von fest in gasförmig oder von flüssig in gasförmig. Die Aggregatszustandsänderung kann durch Temperaturänderung oder durch die Reaktion zweier miteinander reagierender Substanzen hervorgerufen werden. Die Druckbeaufschlagung kann auch nur durch Volumenzunahme eines Mediums mittels eines Pumpmechanismus erfolgen.

Unter dem Term "Druckknopfteil mit Kopf" im Sinne der vorliegenden Erfindung wird insbesondere eine Befestigungsvorrichtung verstanden, welche üblicherweise ein Metallteil umfasst, das mit einem Kopf versehen ist, welcher passend bzw. komplementär zu der Vertiefung eines hierzu komplementären Wirkelementes ist, wobei das komplementäre Wirkelement entweder eine Muffe oder ein Druckknopfteil mit komplementärer Vertiefung ist. Bei einer "Muffe" handelt es sich um ein kurzes rohrförmiges Verbindungsstück.

Unter dem Term "Saugnapf" im Sinne der vorliegenden Erfindung wird insbesondere ein napfförmiges Gebilde oder eine zirkuläre Mulde aus elastischem Material verstanden, das sich mittels Unterdruck an einer glatten Fläche festsaugen kann.

Unter dem Term "Harpunensteg" im Sinne der vorliegenden Erfindung wird insbesondere eine Befestigungsvorrichtung, welche einen ausgebildeten leistenförmigen Vorsprung aufweist, verstanden. Das hierzu komplementäre Wirkelement ist eine Nut, welche eine längliche Vertiefung aufweist, welche komplementär zu dem Vorsprung des Harpunenstegs ist.

Unter dem Term "Clipverbinder" im Sinne der vorliegenden Erfindung wird insbesondere eine Einschnapp-Befestigungsvorrichtung verstanden, die zumindest mit einer Feder versehen ist. Das hierzu komplementäre Wirkelement ist eine "Schwalbenschwanznut". Bei einer "Schwaibenschwanznut" handelt es sich um eine hinterschnittene Nut, bei der die Innenflächen der die Nut begrenzenden Nutschenkel einen spitzen Winkel mit dem Nutgrund bilden. Diese Nutschenkel können von dem komplementären Clipverbinder eingeklemmt werden.

### Aufgabe der Erfindung

Es ist Aufgabe der vorliegenden Erfindung, eine verbesserte gattungsgemäße Vorrichtung zur Verfügung zu stellen, die eine erhöhte Anwenderfreundlichkeit aufweist und auch eine Schmerzreduzierung für den Patienten bei der Behandlung liefert.

### Beschreibung der Erfindung

Die Lösung der Aufgabe ergibt sich aus den Merkmalen des unabhängigen Anspruchs 1.

Demgemäß wird die Aufgabe überraschenderweise durch eine gattungsgemäße Vorrichtung gelöst, wobei am Griffteil ein erstes und am Kopfteil ein zweites gestauchtes Schlauchelement angeordnet ist, welche sowohl am Kopfteil, als auch am Griffteil befestigt sind, und wobei das zweite gestauchte Schlauchelement hohlraumseitig, das erste gestauchte Schlauchelement instrumentenseitig angeordnet ist, und wobei die Länge der gestreckten Schlauchelemente mindestens so lang ist wie die maximale Eindringtiefe.

Die erfindungsgemäße Vorrichtung ist gegenüber dem Stand der Technik vorteilhaft. Bei den aus dem Stand der Technik bekannten Vorrichtungen kommt es immer noch entweder zwischen Außenwand des Instrumentes und Hohlrauminnenwandung, oder zwischen der Innenwandung des Schlauches und Instrumentenaußenseite zu Reibungen. Im Gegensatz dazu bewirkt das erfindungsgemäße mehrlagige Schlauchgebilde, dass die Reibung beim Vorschieben des Instrumentes direkt zwischen das hohlraumseitig angeordnete zweite Schlauchelement des Kopfteils und das instrumentenseitig angeordnete erste Schlauchelement des Griffteils verlegt wird. Somit findet zwischen dem Instrument und dem inneren hohlraumseitig angeordneten zweiten Schlauchelement des Kopfteils und zwischen der Hohlrauminnenwandung und dem äußeren instrumentenseitig angeordneten ersten Schlauchelement des Griffteils keine Relativbewegung statt. Die Reibung der Schlauchelemente findet auf den zueinander gewandten Seiten statt und kann so auf einen möglichst niedrigen Reibungskoeffizienten hin optimiert werden. Der Raum zwischen den beiden aneinander vorbei gleitenden Schlauchelementen ist nach außen geschlossen, so dass keine Feuchtigkeit bzw. Flüssigkeit eindringen kann. Auf diese Weise kann keine Kapillaradhäsion den Gleitwiderstand erhöhen. Aufgrund der vorteilhaften Ausgestaltung der erfindungsgemäßen Vorrichtung wird zudem durch ein Zurückziehen des Griffstückes und damit ein Zurückziehen und "Begradigen" des Hohlraumes ein einfaches Hineinziehen der Vorrichtung in die Kurvaturen des Hohlraumes ermöglicht. Besonders bevorzugt ist es, dass die Länge der gestreckten Schlauchelemente über die maximale Eindringtiefe hinausgeht. Auf diese Weise wird eine "Rückziehreserve" zur Verfügung gestellt, um z.B. in Lage zu sein, bei einem fixierten Koloskop die Schlauchelemente und damit Hohlraumanteile zurückziehen zu können.

Bevorzugt ist eine Verwendung der Vorrichtung für Koloskope. Jedoch ist die Vorrichtung beispielsweise auch für ein Cystoskop, d. h. ein spezielles Endoskop in der Urologie, für ein Bronchoskop, Thorakoskop und Mediastinoskop, d. h. spezielle Endoskope in der Pneumologie, Ösophago-Gastro-Duodeno-Jejunoskopie, d. h. für eine Magen- Darmspiegelung, und für einen peripheren Venenkatheter anwendbar. Bei einer entsprechenden Verwendung kann die empfindlichen Schleimhaut der Harnwege geschützt bzw. der Einstichwiderstand reduziert werden. Die vorliegende Erfindung eignet sich allgemein jedoch zur Einführung eines beliebigen länglichen, zylindrischen Instrumentes in einen Hohlraum.

In einer bevorzugten Ausführungsform der vorliegenden Erfindung ist der wenigstens eine Schlauch ein Folienschlauch. Alternativ kommt beispielsweise auch ein Baumwollschlauch oder ein Seidenschlauch in Betracht. Aus Handhabbarkeitsgründen ist jedoch ein Folienschlauch bevorzugt.

Weiterhin ist bevorzugt, dass der erfindungsgemäße Schlauch einen Durchmesser aufweist, der 1 mm bis 10 mm, vorzugsweise 3 mm bis 7 mm, größer als der maximale Durchmesser des zylindrischen Instruments ist.

In einer bevorzugten Ausführungsform der vorliegenden Erfindung weisen sowohl der Kopfteil, als auch der Griffteil der Vorrichtung eine Länge von ≥ 10 mm bis ≤ 80 mm auf. Die aus Handhabbarkeitsgründen bevorzugte Länge ist von 20 mm bis 40 mm.

Das mehrlagige Schlauchgebilde umfasst bevorzugt zwei Schläuche, kann aber alternativ auch nur einen Schlauch umfassen.

In einer weiteren bevorzugten Ausführungsform der vorliegenden Erfindung ist das zweite gestauchte Schlauchelement sowohl am distalen oder proximalen Ende des Kopfteils, als auch am distalen oder proximalen Ende des Griffteils befestigt und das erste gestauchte Schlauchelement sowohl am distalen oder proximalen Ende des Kopfteils, als auch am distalen oder proximalen Ende des Griffteils befestigt. Alternativ kann die Befestigung jedoch auch irgendwo zwischen den jeweiligen distalen und proximalen Enden erfolgen.

In einer besonders bevorzugten Ausführungsform der vorliegenden Erfindung ist das zweite gestauchte Schlauchelement am distalen Ende des Kopfteils und am proximalen Ende des Griffteils befestigt und das erste gestauchte Schlauchelement am proximalen Ende des Griffteils und am proximalen oder distalen Ende des Kopfteils befestigt. Die Herstellung dieser Ausgestaltung ist am einfachsten durchzuführen.

In einer weiteren besonders bevorzugten Ausführungsform der vorliegenden Erfindung ist das zweite gestauchte Schlauchelement am proximalen Ende des Kopfteils und am proximalen Ende des Griffteils befestigt und das erste gestauchte Schlauchelement am proximalen Ende des Griffteils und am proximalen Ende des Kopfteils befestigt.

Es ist bevorzugt, dass das gestauchte Schlauchelement mittels Adhäsion, insbesondere Klebung, mittels Schweißung, Lötung und/oder Klemmung, insbesondere mittels eines O-Rings, befestigt ist. Auf diese Weise ist eine individuelle Auswahl aus einer Vielzahl möglicher Befestigungsweisen möglich. Die Schweißung kann zum Beispiel mittels Ultraschall, Laserstrahl und/oder Elektronenstrahl erfolgen. Die Lötung kann beispielsweise mit Hilfe eines Laserstrahls erfolgen.

In einer weiteren bevorzugten Ausführungsform der vorliegenden Erfindung enthalten die Schlauchelemente Naturstoffe, beispielsweise Naturkautschuk, Seide- und/oder Baumwollfasern, Kunstfasern aus natürlichen Polymeren, insbesondere Viskosefasern, und/oder Kunststoffe, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydrierten Acrylnitrilbutadien-Kautschuk, Polyethylenterephthalat, Polyethylen, insbesondere hochmolekulares und/oder ultrahochmolekulares Polyethylen, und ggf. Zusätze, beispielsweise Puder, Silikonöl, Wachs, Mikroballons, Molybdänsulfid, Graphit, Talkum, Teflonpulver und/oder Selbstschmierungs-Additive, insbesondere Polyacetal, Polyphenylensulfid, Polyphthalamid und/oder Polyetheretherketon, und ggf. eine Beschichtung, beispielsweise mittels Teflon und/oder eine metallisierte Beschichtung: Auf diese Weise ist eine individuelle Auswahl aus einer Vielzahl möglicher Zusammensetzungen möglich. Ferner kann durch den Einsatz einer Beschichtung der Reibungswiderstand noch zusätzlich verringert werden.

Die Stauchung kann chaotisch oder definiert, insbesondere chaotisch, sein. Stauchung bedeutet dabei jede beliebige Faltung bzw. Knitterung, um den Schlauch raumsparend unterzubringen. Diese chaotische Ausgestaltung ist bevorzugt, da sie am einfachsten herzustellen ist.

Die Einführung kann grundsätzlich auf unterschiedliche Weise erfolgen. Ein Vorteil bei der Verwendung der erfindungsgemäßen Vorrichtung ist, dass eine Zuhilfenahme von Hilfsmitteln nicht erforderlich ist. Dennoch kann die erfindungsgemäße Vorrichtung alternativ mindestens ein Hilfsmittel zur Einführung des Instruments aufweisen, wobei das mindestens eine Hilfsmittel beispielsweise ein mechanisches, hydraulisches oder pneumatisches Hilfsmittel ist. Vorzugsweise ist mindestens eine ggf. umhüllte Substanz an der Innenseite der Schlauchelemente angeordnet, welche beispielsweise durch Erwärmung verdampft oder sublimiert. So längt sich bei einer Erwärmung der Schlauch und wird in den zu untersuchenden Hohlraum eingeführt. Eine weitere Alternative besteht darin, dass mindestens zwei separat umhüllte flüssige und/oder feste Substanzen an der Innenseite der Schlauchelemente angeordnet sind, welche bei Inkontaktbringung unter Volumenzunahme miteinander zu einem Gas reagieren. Die Wirkung wäre entsprechend der erstgenannten Alternative. Entsprechend würde eine einfache Druckbeaufschlagung von außen an die Innenseite der Schlauchelemente wirken. Alternativ kann die Vorrichtung am Griffstück als mechanisches Hilfsmittel eine motorisch betriebene Einzugsvorrichtung aufweisen und/oder ankoppelbar sein. Bei der zuerst genannten Alternative ist vorgesehen, dass die Schlauchelemente mechanisch transportiert werden, beispielsweise durch Wellen bzw. Rollen. Dabei werden über ein Getriebe zwei gegenläufige Wellen bzw. Förderrollen angetrieben. Die Vorrichtung liegt hierbei mittig. Auf diesen Wellen bzw. Förderrollen können die Schlauchelemente aufgewickelt werden, welche dann im Motorbereich irreversibel in zwei Teile aufgetrennt werden. Auf diese Weise können die Schlauchelemente über das Instrument gezogen werden. Dieser Einzug kann so lange fortgesetzt werden, bis der Schlauchvorrat aufgebraucht ist.

Um ein vorzeitiges Loslösen bzw. Abstreifen des gestauchten Schlauchelementes von der Vorrichtung bei der Einführung zu verhindern, ist es bevorzugt, dass eine Schutzfolie über dem gestauchten Schlauchelement vorgesehen ist.

In einer bevorzugten Ausführungsform der vorliegenden Erfindung enthält die Schutzfolie Naturstoffe, beispielsweise Naturkautschuk, Seide- und/oder Baumwollfasern, Kunstfasern aus natürlichen Polymeren, insbesondere Viskosefasern, und/oder Kunststoffe, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydrierten Acrylnitrilbutadien-Kautschuk, Polyethylenterephthalat, Polyethylen, insbesondere hochmolekulares und/oder ultrahochmolekulares Polyethylen, und ggf. Zusätze, beispielsweise Puder, Silikonöl, Wachs, Mikroballons, Molybdänsulfid, Graphit, Talkum, Teflonpulver und/oder Selbstschmierungs-Additive, insbesondere Polyacetal, Polyphenylensulfid, Polyphthalamid und/oder Polyetheretherketon, und ggf. eine Beschichtung, beispielsweise mittels Teflon und/oder eine metallisierte Beschichtung. Auf diese Weise ist eine individuelle Auswahl aus einer Vielzahl möglicher Zusammensetzungen möglich. Ferner kann durch den Einsatz einer Beschichtung der Reibungswiderstand noch zusätzlich verringert werden.

Bevorzugt ist dabei, dass die erfindungsgemäße Schutzfolie als flexibler Folienschlauch, Überschlauch, Schrumpfschlauch und/oder als starre Hülse vorgesehen ist.

In einer besonders bevorzugten Ausführungsform der vorliegenden Erfindung ist die Schutzfolie über dem Kopfteil übergestülpt. Diese Ausgestaltung ist am einfachsten herzustellen.

Alternativ kann die Schutzfolie am distalen oder proximalen Ende des Kopfteils befestigt sein. Am bevorzugtesten ist dabei, dass die Schutzfolie aus dem distalen Ende des zweiten gestauchten Schlauchelementes geformt ist. Die Schutzfolie des Griffteils kann ebenfalls aus der Schutzfolie bzw. dem Folienschlauch des Kopfteils bestehen.

Ebenfalls bevorzugt ist eine Ausführungsform der vorliegenden Erfindung, wobei über dem zweiten gestauchten Schlauchelement eine Schutzhülse und/oder über dem ersten gestauchten Schlauchelement eine Schutzhülse vorgesehen ist. Auch diese Ausgestaltung verhindert ein vorzeitiges Loslösen bzw. Abstreifen des gestauchten Schlauchelementes von der Vorrichtung beim Einführen des Instrumentes. Besonders bevorzugt ist dabei, dass die Schutzhülse des Kopfteils am proximalen Ende des Kopfteils befestigt ist, und die Schutzhülse des Griffteils am proximalen Ende des Griffteils befestigt ist.

In dem Fall, dass das zweite gestauchte Schlauchelement am proximalen Ende des Kopfteils befestigt und distal umgeschlagen ist, wirkt das umgeschlagene Schlauchelement selbst als Schutzfolie, wodurch die zusätzliche Verwendung einer Schutzfolie bzw. Schutzhülse nicht erforderlich ist.

In einer weiteren bevorzugten Ausführungsform der vorliegenden Erfindung ist im Auslieferungszustand eine Schutzkappe über dem distalen Ende des Kopfteils gestülpt. Diese Schutzkappe verhindert ein vorzeitiges Loslösen bzw. Abstreifen des gestauchten Schlauchelementes von der Vorrichtung vor der Anwendung, beispielsweise beim Transport, und wird vor der Anwendung entfernt.

Vorzugsweise ist die Vorrichtung an dem distalen Ende des Instrumentes, an dem proximalen Ende des beweglichen Teils des Instrumentes oder auf dem beweglichen Teil des Instrumentes befestigt und ggf. selbst biegeelastisch. In einer besonders bevorzugten Ausführungsform ist die Vorrichtung am distalen Ende des Instrumentes befestigt und mindestens zum Teil biegeelastisch. Diese Ausführungsform ist am einfachsten zu handhaben und behindert die Beweglichkeit des Instrumentes am wenigsten.

Bevorzugt ist eine Fixierung der erfindungsgemäßen Vorrichtung an das längliche, zylindrische Instrument. Die Vorrichtung kann dabei beispielsweise auf das Instrument aufgeschoben, aufgeklemmt, eingehakt, festgesaugt, angeheftet, insbesondere aufgeklebt, oder mittels Gewinde aufgedreht werden. In der vorliegenden Erfindung ist die Befestigung der Vorrichtung an das Instrument vorzugsweise mittels eines Gewindes, einer Klemme, insbesondere eines Clipverbinders, mittels eines Gummibandes, eines Magneten, eines Raffbandes, eines Klettbandes, eines Harpunensteges, eines Druckknopfes, eines Saugnapfes, einer zirkulären Sauglippe, eines Gummirings, eines Bajonettverschlusses, einer Druckmanschette und/oder eines Adhäsionsmittels, insbesondere eines lösbaren Klebemittels, beispielsweise mittels eines Klebebandes und/oder eines Kleberings, und eines mechanischen Anschlags, eines Drehrings und/oder einer starren oder elastischen Aufsatzkappe vorgesehen. Diese Befestigungsvorrichtungen haben den Vorteil, dass sie reversibel befestigt werden können, d. h. dass die Befestigung jederzeit wieder gelöst werden kann, und somit eine Entfernung ermöglicht wird, die ohne einen größeren Aufwand, auf einfache Art und Weise und/oder ohne Spuren zu hinterlassen, erfolgen kann. Es kann auch jede andere reversible Befestigungsvorrichtung eingesetzt werden. Dabei ist jeweils, wenn nötig, an der Vorrichtung bzw. an dem länglichen Instrument ein zu der Befestigungsvorrichtung entsprechendes komplementäres Wirkelement angeordnet. Ein komplementäres Wirkelement für einen Magneten kann z.B. eine magnetische Oberfläche oder ein komplementärer Magnet sein. Komplementäre Wirkelemente für Klebebänder, Kleberinge und/oder Saugnäpfe können beispielsweise glatte Oberflächen sein, da diese so ausgestaltet sind, dass Klebebänder, Kleberinge und/oder Saugnäpfe reversibel an diese anbringbar sind. Ein komplementäres Wirkelement für einen Druckknopfteil mit Kopf ist ein Druckknopfteil mit Vertiefung und/oder eine Muffe, für einen Harpunensteg eine Nut und für einen Clipverbinder eine Schwalbenschwanznut. Ein komplementäres Wirkelement für ein Klettband und/oder eine Druckmanschette ist ein Flauschband. Ferner kann auch der Einsatz eines mechanischen Anschlags, eines Drehrings und/oder einer Aufsatzkappe vorgesehen sein, da diese Befestigungselemente besonders anwenderfreundlich sind. Diese Elemente sind vorzugsweise in Verbindung mit mindestens einer weiteren offenbarten Befestigungsvorrichtung vorgesehen. So wird ein mechanischer Anschlag bevorzugt in Verbindung mit einer Klemmvorrichtung und/oder einem Klebeelement verwendet. Ein Drehring gemäß der vorliegenden Erfindung ist vorzugsweise am distalen Teil der Vorrichtung angeordnet und weist auf seiner Innenfläche ein konisches Innengewinde auf, während die Außenfläche der Klemmvorrichtung mit einem entsprechenden konischen Außengewinde versehen ist, wodurch durch Drehung des Drehringes eine Durchmesserverkleinerung der Klemmvorrichtung erzielt wird, welche die Fixierung der Klemmvorrichtung auf dem Instrument bewirkt. Eine Aufsatzkappe gemäß der vorliegenden Erfindung, welche vorzugsweise am distalen Kopfteil der Vorrichtung angeordnet ist, umfasst bevorzugt ein elastisches Material und/oder wird bevorzugt in Verbindung mit einem mechanischen Anschlag zur Befestigung an dem länglichen Instrument verwendet. Auf diese Weise ist eine individuelle Auswahl aus einer Vielzahl möglicher Befestigungsvarianten möglich. Ferner ist so eine genaue Positionierung der Vorrichtung an dem Instrument möglich, und aufwendige Justierprozesse werden vermieden.

Alternativ kann die Vorrichtung jedoch auch in das Instrument direkt integriert sein.

Bevorzugt ist, dass der Kopfteil in der Startposition partiell auf dem Griffteil fixiert bzw. festgesteckt oder der Kopfteil mit dem Griffteil mittels Sollbruchstelle verbunden ist. Bei Beginn der Untersuchung kann die Fixierung so auf einfache Weise aufgehoben werden.

Vorzugsweise weist die Vorrichtung ein ergonomisches Griffstück, ein Griffstück in Revolverabzugsform, ein Griffstück in Schlaufenform und/oder ein Griffstück mit Schlaufe auf. Mittels dieser Ausgestaltungen ist eine einfachere und bequemere Handhabung für den Anwender möglich. Das Griffstück ist dabei bevorzugt so ausgebildet, dass es beim Vorschieben nicht inkorporiert wird, und ist außerdem so kurz geformt, dass am Ende der Behandlung, wenn das Instrument in seiner gesamten Länge eingeführt ist, möglichst wenig funktionelle Länge des Instrumentes durch das Griffstück blockiert wird. Besonders bevorzugt ist ein Griffstück, das ein oder zwei Flügelpaare umfasst oder rotationssymetrisch wie ein Trichter geformt ist. Die Flügel, die vorzugsweise in abgewinkelter Form vorliegen, können beispielsweise im 90° und/oder 45° Winkel angeordnet sein. Alternativ können die Flügel bzw. der Trichter eine Doppelwinkelform aufweisen.

In einer weiteren bevorzugten Ausführungsform der vorliegenden Erfindung weist der erfindungsgemäße Griffteil bzw. das Griffstück zusätzlich einen "Mitnehmer" auf. Dieser kann beim Zurückziehen den Griffteil bzw. das Griffstück "mitnehmen", so dass sich der entfaltete Folienschlauch nicht im Hohlraum aufbauschen und ein Hindernis bilden kann. Der Mitnehmer kann beispielsweise eine gelochte Gummiplatte oder ein selbstklemmender Gummiring sein. Durch den Einsatz eines Mitnehmers wird die Haftreibung erhöht. Auf diese Weise kann beim Zurückziehen des Instrumentes nach der Anwendung auch der entfaltete Schlauch leichter mit zurück gezogen werden.

Weiterhin ist bevorzugt, dass die erfindungsgemäße Vorrichtung eine teilweise oder vollständig Ummantelung, beispielsweise mit Hilfe von Naturstoffen, beispielsweise Naturkautschuk, Seide- und/oder Baumwollfasern, Kunstfasern aus natürlichen Polymeren, insbesondere Viskosefasern, und/oder Kunststoffen, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydriertem Acrylnitrilbutadien-Kautschuk, Polyethylenterephthalat, Polyethylen, insbesondere hochmolekularem und/oder ultrahochmolekularem Polyethylen, und ggf. Zusätzen, beispielsweise Puder, Silikonöl, Wachs, Mikroballons, Molybdänsulfid, Graphit, Talkum, Teflonpulver und/oder Selbstschmierungs-Additiven, insbesondere Polyacetal, Polyphenylensulfid, Polyphthalamid und/oder Polyetheretherketon, und ggf. einer Beschichtung, beispielsweise mittels Teflon und/oder einer metallisierten Beschichtung, Glas und/oder Plexiglas, insbesondere Polyurethan und/oder Polymethylmethacrylat, aufweist.

Die Vorrichtung ist dabei teilweise oder vollständig hermetisch abgeschlossen, wobei besonders bevorzugt ist, dass der distale Bereich des Kopfteils von einer transparenten Hülle bzw. Kappe umgeben ist. Die Optik des Instrumentes ist dabei durch die transparente Hülle bzw. Kappe nicht beeinträchtigt. Durch die erfindungsgemäße Ausgestaltung wird das Instrument selbst nicht oder nur vermindert kontaminiert. Bei einem vollständigen Hygieneschutz muss das Instrument vor einer Untersuchung nicht mehr desinfiziert werden und kann nach der Untersuchung und Entfernung der Vorrichtung ohne längere Verzögerungen wieder eingesetzt werden, sobald eine neue erfindungsgemäße Vorrichtung befestigt wurde. Auf diese Weise ist eine schnellere Anwendung möglich. Dabei ist bevorzugt vorgesehen, dass ein Teil der üblicherweise in dem Instrument angeordneten Kanäle, wie zum Beispiel der Luftkanal, die Waschdüse und ggf. auch der Arbeitskanal bzw. Instrumentenkanal, vor einer Anwendung zusammen mit der erfindungsgemäßen Hilfsvorrichtung an dem Instrument befestigt wird. Diese Kanäle befinden sich vorzugsweise außenseitig an dem Instrument, sind mit dem distalen Ende der Vorrichtung verbunden und münden dort im Bereich der transparenten Hülle bzw. Kappe über Durchtrittsöffnungen nach außen.

Im Fall eines teilweisen Hygieneschutzes ist es bevorzugt, dass die entsprechenden Kanalschläuche außenseitig oder in außenseitigen Nuten bzw. Rillen des Instrumentes auswechselbar angeordnet sind und/oder in Durchtrittsöffnungen der transparenten Hülle bzw. Kappe münden. Nach dem Abschluss einer Untersuchung können die Kanalschläuche und die Vorrichtung vollständig entfernt und ausgewechselt werden.

In einer weiteren bevorzugten Ausführungsform der vorliegenden Erfindung ist die Vorrichtung als Einmalartikel vorgesehen.

Die vorgenannten sowie die beanspruchten und in den Zeichnungen beschriebenen erfindungsgemäß zu verwendenden Komponenten unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

### Ausführungsbeispiele und Zeichnungen

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

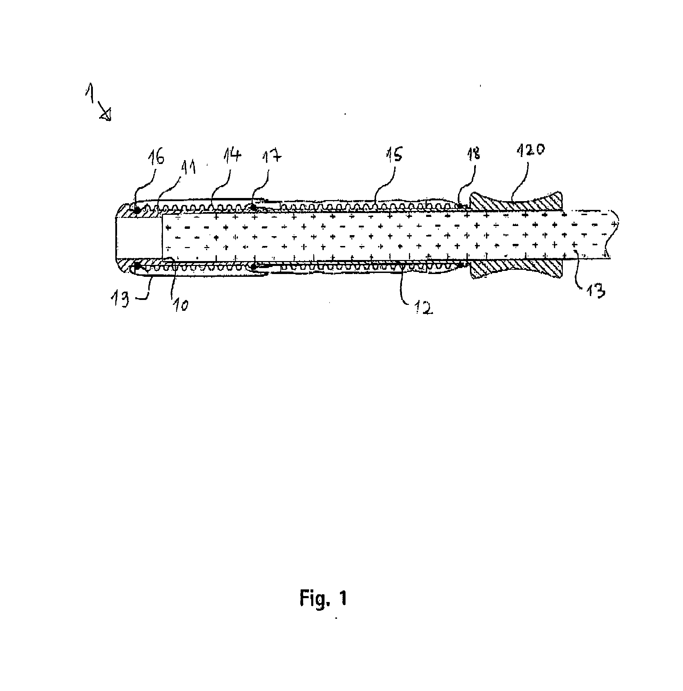

**Fig. 1** zeigt eine Vorrichtung **1,** die einen distalen Kopfteil **11** und einen proximalen Griffteil **12** aufweist. Das zweite gestauchte Folienschlauchelement **14** ist am distalen Ende des Kopfteils **11** mittels O-Ring **16** befestigt und am proximalen Ende des Griffteils **12** mittels O-Ring **18.** Das erste gestauchte Folienschlauchelement **15** ist am proximalen Ende des Griffteils **12** mittels O-Ring **18** befestigt und am proximalen Ende des Kopfteils **11** mittels O-Ring **17.** Das zweite gestauchte Folienschlauchelement **14** ist darmseitig und das erste gestauchte Folienschlauchelement **15** ist koloskopseitig angeordnet. Bei dem zweilagigen Folienschlauchgebilde wird die Reibung beim Vorschieben des Koloskops **13** direkt zwischen das darmseitig angeordnete zweite Folienschlauchelement **14** und das koloskopseitig angeordnete erste Folienschlauchelement **15** verlegt. Über dem zweiten gestauchten Folienschlauchelement **14** ist eine Schutzfolie **19** angeordnet, wodurch ein vorzeitiges Loslösen bzw. ungeordnetes Abstreifen des Folienschlauches **14** von der Vorrichtung 1 bei der Einführung verhindert wird. Die Schutzfolie **19** ist mittels O-Ring **16** am distalen Ende des Kopfteils **11** befestigt und umschließt mittels Eigenelastizität das zweite gestauchte Folienschlauchelement **14** am proximalen Ende des Kopfteils **11.** Die Vorrichtung 1 wird mittels eines mechanischen Anschlags **10** an dem Koloskop **13** an einem definierten Punkt fixiert. Am Griffteil **12** ist das Griffstück **120** angeordnet.

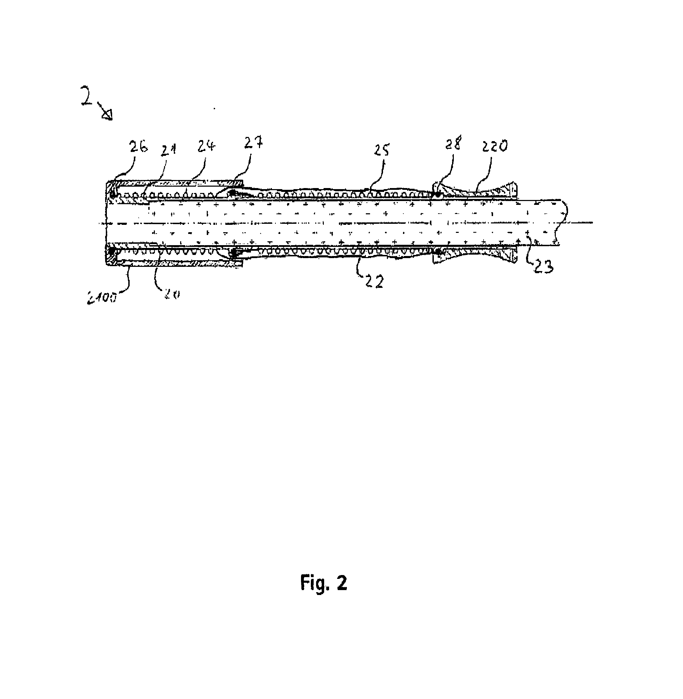

**Fig. 2** zeigt eine Vorrichtung **2,** die einen distalen Kopfteil **21** und einen proximalen Griffteil **22** aufweist. Das zweite gestauchte Folienschlauchelement **24** ist am distalen Ende des Kopfteils **21** mittels O-Ring **26** befestigt und am proximalen Ende des Griffteils **22** mittels O-Ring **28.** Das erste gestauchte Folienschlauchelement **25** ist am proximalen Ende des Griffteils **22** mittels O-Ring **28** befestigt und am proximalen Ende des Kopfteils **21** mittels O-Ring **27.** Das zweite gestauchte Folienschlauchelement **24** ist darmseitig und das erste gestauchte Folienschlauchelement **25** ist koloskopseitig angeordnet. Bei dem zweilagigen Folienschlauchgebilde wird die Reibung beim Vorschieben des Koloskops **23** direkt zwischen das darmseitig angeordnete zweite Folienschlauchelement **24** und das koloskopseitig angeordnete erste Folienschlauchelement **25** verlegt. Über dem zweiten gestauchten Folienschlauchelement **24** ist eine Schutzhülse **2100** angeordnet, welche ein vorzeitiges Loslösen bzw. Abstreifen des zweiten Folienschlauches **24** von der Vorrichtung **2** bei der Einführung verhindert. Die Schutzhülse **2100** des Kopfteils ist am distalen Ende des Kopfteils **21** befestigt Die Vorrichtung **2** wird mittels eines mechanischen Anschlags **20** an einem definierten Punkt an dem Koloskop **23** fixiert. Am Griffteil **22** ist das Griffstück **220** angeordnet.

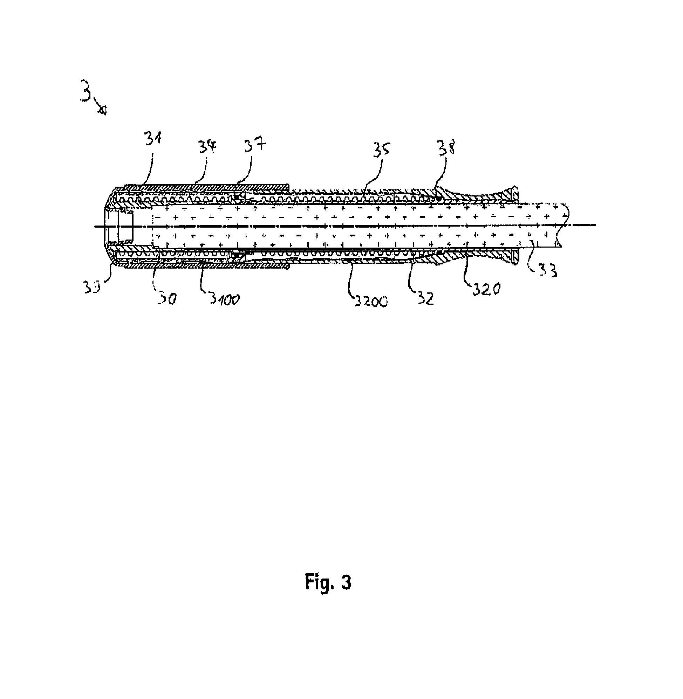

**Fig. 3** zeigt eine Vorrichtung **3,** die einen distalen Kopfteil **31** und einen proximalen Griffteil **32** aufweist. Das zweite gestauchte Folienschlauchelement **34** ist am proximalen Ende des Kopfteils **31** mittels O-Ring **37** befestigt und am proximalen Ende des Griffteils **32** mittels O-Ring **38.** Das erste gestauchte Folienschlauchelement **35** ist am proximalen Ende des Griffteils **32** mittels O-Ring **38** befestigt und am proximalen Ende des Kopfteils **31** mittels O-Ring **37.** Das zweite gestauchte Folienschlauchelement **34** ist darmseitig und das erste gestauchte Folienschlauchelement **35** ist koloskopseitig angeordnet. Bei dem zweilagigen Folienschlauchgebilde wird die Reibung beim Vorschieben des Koloskops **33** direkt zwischen das darmseitig angeordnete zweite Folienschlauchelement **34** und das koloskopseitig angeordnete erste Folienschlauchelement **35** verlegt. Über dem zweiten gestauchten Folienschlauchelement **34** ist eine Transportschutzkappe **39** angeordnet, wodurch ein vorzeitiges Loslösen bzw. Abstreifen des zweiten Folienschlauches **34** von der Vorrichtung **3** vor der Anwendung verhindert wird. Sowohl über dem zweiten gestauchten Folienschlauchelement **34,** als auch über dem ersten gestauchten Folienschlauchelements **35** ist eine Schutzhülse **3100** bzw. **3200** angeordnet, welche ein vorzeitiges Loslösen bzw. Abstreifen des Folienschlauches **34** bzw. **35** von der Vorrichtung 3 bei der Einführung verhindern. Die Schutzhülse **3100** des Kopfteils ist am proximalen Ende des Kopfteils **31** befestigt, und die Schutzhülse **3200** des Griffteils ist am proximalen Ende des Griffteils **32** befestigt. Am proximalen Ende der Schutzhülse **3200** ist das Griffstück **320** angeordnet. Die Vorrichtung **3** wird mittels eines mechanischen Anschlags **30** an einem definierten Punkt an dem Koloskop **33** fixiert.

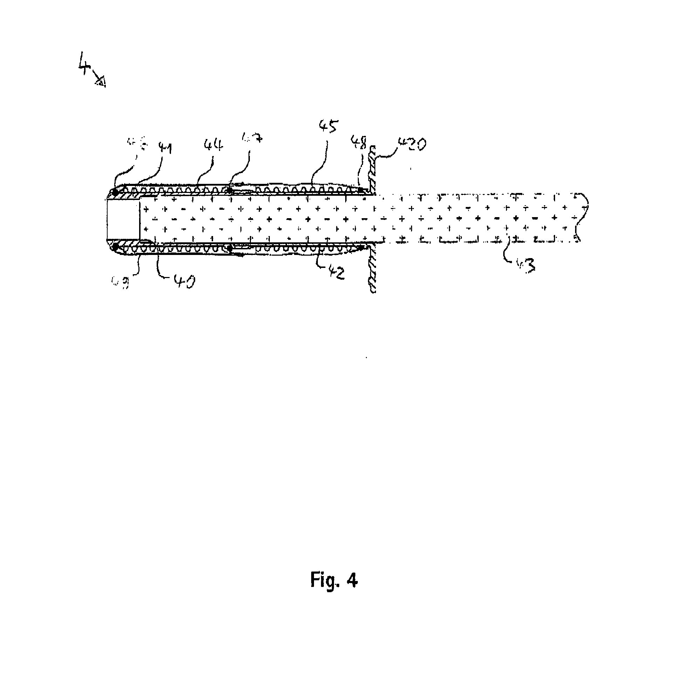

**Fig. 4** zeigt eine Vorrichtung **4**, die einen distalen Kopfteil **41** und einen proximalen Griffteil **42** aufweist. Das zweite gestauchte Folienschlauchelement **44** ist am distalen Ende des Kopfteils **41** mittels O-Ring **46** befestigt und am proximalen Ende des Griffteils **42** mittels O-Ring **48.** Das erste gestauchte Folienschlauchelement **45** ist am proximalen Ende des Griffteils **42** mittels O-Ring **48** befestigt und am proximalen Ende des Kopfteils **41** mittels O-Ring **47.** Das zweite gestauchte Folienschlauchelement **44** ist darmseitig und das erste gestauchte Folienschlauchelement **45** ist koloskopseitig angeordnet. Bei dem zweilagigen Folienschlauchgebilde wird die Reibung beim Vorschieben des Koloskops **43** direkt zwischen das darmseitig angeordnete zweite Folienschlauchelement **44** und das koloskopseitig angeordnete erste Folienschlauchelement **45** verlegt. Über dem zweiten gestauchten Folienschlauchelement **44** ist eine Schutzfolie **49** angeordnet, wodurch ein vorzeitiges Loslösen bzw. Abstreifen des zweiten Folienschlauches **44** von der Vorrichtung **4** bei der Einführung verhindert wird. Die Schutzfolie **49** ist mittels O-Ring **46** am distalen Ende des Kopfteils **41** befestigt und umschließt mittels Eigenelastizität das zweite gestauchte Folienschlauchelement **44** am proximalen Ende des Kopfteils **41.** Die Vorrichtung **4** wird mittels eines mechanischen Anschlags **40** an dem Koloskop **43** an einem definierten Punkt fixiert. Am Griffteil **42** ist am proximalen Ende das Griffstück **420** angeordnet. Das Griffstück **420** umfasst ein Flügelpaar, wobei die Flügel im 90° Winkel angeordnet sind.

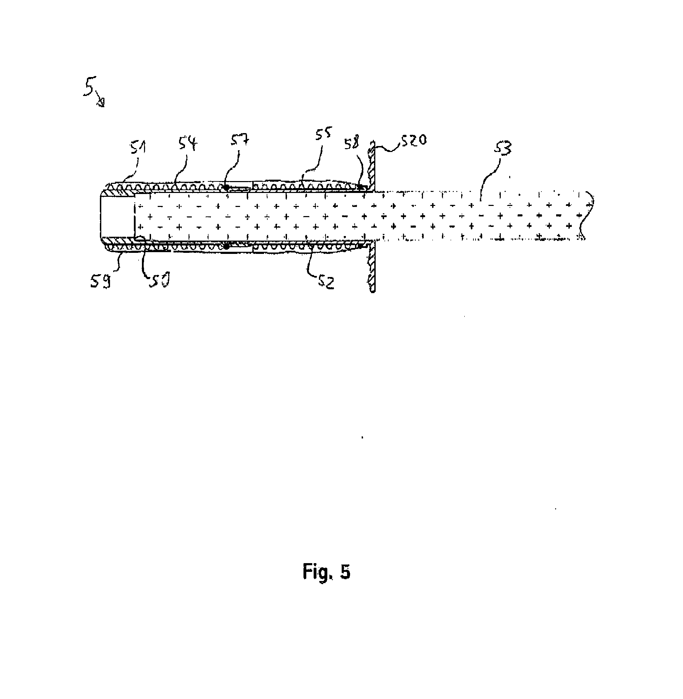

**Fig. 5** zeigt eine Vorrichtung **5,** die einen distalen Kopfteil **51** und einen proximalen Griffteil **52** aufweist. Das zweite gestauchte Folienschlauchelement **54** ist am proximalen Ende des Kopfteils **51** mittels O-Ring **57** befestigt und am proximalen Ende des Griffteils **52** mittels O-Ring **58.** Das erste gestauchte Folienschlauchelement **55** ist am proximalen Ende des Griffteils **52** mittels O-Ring **58** befestigt und am proximalen Ende des Kopfteils **51** mittels O-Ring **57**. Das zweite gestauchte Folienschlauchelement **54** ist am distaeln Ende des Kopfteils **51** umgeschlagen und wirkt somit selbst als Schutzfolie **59,** wodurch die zusätzliche Verwendung einer weiteren Schutzfolie bzw. Schutzhülse nicht erforderlich ist. Das zweite gestauchte Folienschlauchelement **54** ist darmseitig und das erste gestauchte Folienschlauchelement **55** ist koloskopseitig angeordnet. Bei dem zweilagigen Folienschlauchgebilde wird die Reibung beim Vorschieben des Koloskops **53** direkt zwischen das darmseitig angeordnete zweite Folienschlauchelement **54** und das koloskopseitig angeordnete erste Folienschlauchelement **55** verlegt. Die Folienschlauchelemente **54** und **55** gehören zu demselben Folienschlauch. Die Vorrichtung **5** wird mittels eines mechanischen Anschlags **50** an einem definierten Punkt an dem Koloskop 53 fixiert. Am proximalen Ende des Griffteils **52** ist das Griffstück **520** angeordnet. Das Griffstück **520** umfasst ein Flügelpaar, wobei die Flügel im 90° Winkel angeordnet sind.

## Patentansprüche

1. Vorrichtung **(1, 2, 3, 4, 5)** zur Einführung eines daran befestigbaren oder integrierbaren länglichen, zylindrischen Instrumentes **(13, 23, 33, 43, 53),** insbesondere eines flexiblen Endoskops, in einen Hohlraum mit
einem proximalen Griffteil **(12, 22, 32, 42, 52),**
einem distalen Kopfteil **(11, 21, 31, 41, 51)** und
einem mehrlagigen wenigsten aus einem Schlauch bestehendem Schlauchgebilde, **dadurch gekennzeichnet, dass**
am Griffteil **(12, 22, 32, 42, 52)** ein erstes und am Kopfteil **(11, 21, 31, 41, 51)** ein zweites gestauchtes Schlauchelement **(15, 25, 35, 45, 55, 14, 24, 34, 44, 54)** angeordnet ist, welche sowohl am Kopfteil **(11, 21, 31, 41, 51),** als auch am Griffteil **(12, 22, 32, 42, 52)** befestigt sind, und wobei
das zweite gestauchte Schlauchelement **(14, 24, 34, 44, 54)** hohlraumseitig,
das erste gestauchte Schlauchelement **(15, 25, 35, 45, 55)** instrumentenseitig angeordnet ist, und
wobei die Länge der gestreckten Schlauchelemente **(15, 25, 35, 45, 55, 14, 24, 34, 44, 54)** mindestens so lang ist wie die maximale Eindringtiefe.

2. Vorrichtung **(1, 2, 3, 4, 5)** gemäß Anspruch 1, **dadurch gekennzeichnet, dass** der wenigstens eine Schlauch ein Folienschlauch ist.

3. Vorrichtung **(1, 2, 3, 4, 5)** gemäß Anspruch 1 oder 2, **dadurch gekennzeichnet, dass** der Schlauch einen Durchmesser aufweist, der 1 mm bis 10 mm, vorzugsweise 3 mm bis 7 mm, größer als der maximale Durchmesser des zylindrischen Instruments ist.

4. Vorrichtung **(1, 2, 3, 4, 5)** gemäß einem der vorhergehenden Ansprüche, **dadurch gekennzeichnet, dass** sowohl der Kopfteil **(11, 21, 31, 41, 51),** als auch der Griffteil **(12, 22, 32, 42, 52)** eine Länge von ≥ 10 mm bis ≤ 80 mm, insbesondere eine Länge von 20 mm bis 40 mm, aufweisen.

5. Vorrichtung **(1, 2, 3, 4, 5)** gemäß einem der vorhergehenden Ansprüche, **dadurch gekennzeichnet, dass**
das zweite gestauchte Schlauchelement **(14, 24, 34, 44, 54)** sowohl am distalen oder proximalen Ende des Kopfteils **(11, 21, 31, 41, 51),** als auch am distalen oder proximalen Ende Griffteils **(12, 22, 32, 42, 52)** befestigt ist, und
das erste gestauchte Schlauchelement **(15, 25, 35, 45, 55)** sowohl am distalen oder proximalen Ende des Kopfteils **(11, 21, 31, 41, 51),** als auch am distalen oder proximalen Ende des Griffteils **(12, 22, 32, 42, 52)** befestigt ist.

6. Vorrichtung **(1, 2, 4)** gemäß einem der vorhergehenden Ansprüche, **dadurch gekennzeichnet, dass**
das zweite gestauchte Schlauchelement **(14, 24, 44)** am distalen Ende des Kopfteils **(11, 21, 41)** und am proximalen Ende des Griffteils **(12, 22, 42)** befestigt ist, und
das erste gestauchte Schlauchelement **(15, 25, 45)** am proximalen Ende des Griffteils **(12, 22, 42)** und am proximalen oder distalen Ende des Kopfteils **(11, 21, 41** befestigt ist.

7. Vorrichtung **(1, 2, 3, 4, 5)** gemäß einem der vorhergehenden Ansprüche, **dadurch gekennzeichnet, dass** das gestauchte Schlauchelement mittels Adhäsion, insbesondere Klebung, mittels Schweißung, Lötung und/oder Klemmung, insbesondere mittels eines O-Rings **(16, 17, 18, 26, 27, 28, 37, 38, 46, 47, 48, 57, 58),** befestigt ist.

8. Vorrichtung **(1, 2, 3, 4, 5)** gemäß einem der vorhergehenden Ansprüche, **dadurch gekennzeichnet, dass** die Schlauchelemente Naturstoffe, beispielsweise Naturkautschuk, Seide- und/oder Baumwollfasern, Kunstfasern aus natürlichen Polymeren, insbesondere Viskosefasern, und/oder Kunststoffe, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydrierten Acrylnitrilbutadien-Kautschuk, Polyethylenterephthalat, Polyethylen, insbesondere hochmolekulares und/oder ultrahochmolekulares Polyethylen, und ggf. Zusätze, beispielsweise Puder, Silikonöl, Wachs, Mikroballons, Molybdänsulfid, Graphit, Talkum, Teflonpulver und/oder Selbstschmierungs-Additive, insbesondere Polyacetal, Polyphenylensulfid, Polyphthalamid und/oder Polyetheretherketon, und ggf. eine Beschichtung, beispielsweise mittels Teflon und/oder eine metallisierte Beschichtung, enthalten.

9. Vorrichtung **(1, 2, 3, 4, 5)** gemäß einem der vorhergehenden Ansprüche, **dadurch gekennzeichnet, dass** die Stauchung chaotisch oder definiert ist.

10. Vorrichtung **(1, 2, 3, 4, 5)** gemäß einem der vorhergehenden Ansprüche, **dadurch gekennzeichnet, dass** sie mindestens ein Hilfsmittel zur Einführung des Instruments aufweist, wobei das mindestens eine Hilfsmittel beispielsweise ein mechanisches oder hydraulisches Hilfsmittel ist.

11. Vorrichtung **(1, 4, 5)** gemäß einem der vorhergehenden Ansprüche, **dadurch gekennzeichnet, dass** eine Schutzfolie **(19, 49, 59)** über dem gestauchten Schlauchelement **(14, 44, 54; 15, 45, 55)** vorgesehen ist.

12. Vorrichtung **(1, 4, 5)** gemäß einem der vorhergehenden Ansprüche, **dadurch gekennzeichnet, dass** die Schutzfolie **(19, 49, 59)** Naturstoffe, beispielsweise Naturkautschuk, Seide- und/oder Baumwollfasern, Kunstfasern aus natürlichen Polymeren, insbesondere Viskosefasern, und/oder Kunststoffe, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydrierten Acrylnitrilbutadien-Kautschuk, Polyethylenterephthalat, Polyethylen, insbesondere hochmolekulares und/oder ultrahochmolekulares Polyethylen, und ggf. Zusätze, beispielsweise Puder, Silikonöl, Wachs, Mikroballons, Molybdänsulfid, Graphit, Talkum, Teflonpulver und/oder Selbstschmierungs-Additive, insbesondere Polyacetal, Polyphenylensulfid, Polyphthalamid und/oder Polyetheretherketon, und ggf. eine Beschichtung, beispielsweise mittels Teflon und/oder eine metallisierte Beschichtung, enthält.

13. Vorrichtung gemäß einem der vorhergehenden Ansprüche, **dadurch gekennzeichnet, dass** die Schutzfolie über dem Kopfteil übergestülpt ist.

14. Vorrichtung **(1, 4, 5)** gemäß einem der vorhergehenden Ansprüche, **dadurch gekennzeichnet, dass** die Schutzfolie **(19, 49, 59)** am distalen oder proximalen Ende des Kopfteils **(11, 41, 51)** befestigt ist.

15. Vorrichtung **(2, 3)** gemäß einem der vorhergehenden Ansprüche, **dadurch gekennzeichnet, dass** über dem zweiten gestauchten Schlauchelement **(24, 34)** eine Schutzhülse **(2100, 3100)** und/oder über dem ersten gestauchten Schlauchelement **(35)** eine Schutzhülse **(3200)** vorgesehen ist.

16. Vorrichtung **(3)** gemäß einem der vorhergehenden Ansprüche, **dadurch gekennzeichnet, dass** im Auslieferungszustand eine Schutzkappe **(39)** über dem distalen Ende des Kopfteils **(31)** gestülpt ist.

17. Vorrichtung **(1, 2, 3, 4, 5)** gemäß einem der vorhergehenden Ansprüche, **dadurch gekennzeichnet, dass** sie eine teilweise oder vollständige Ummantelung, beispielsweise mit Hilfe von Naturstoffen, beispielsweise Naturkautschuk, Seide- und/oder Baumwollfasern, Kunstfasern aus natürlichen Polymeren, insbesondere Viskosefasern, und/oder Kunststoffen, beispielsweise Polyvinylchlorid, Ethylen-Vinylalkohol, Polynorbornen-Kautschuk, Polyoctenamer, Acrylnitril-Chloropren-Kautschuk, Polypropylen, Polyvinylidenchlorid, Polystyrol, Polyamid, insbesondere Polyhexamethylenadipinsäureamid, Paraphenylendiamin und/oder Terephthaloyldichlorid, Polycarbonat, Isopren-Kautschuk, Butadien-Kautschuk, Chloropren-Kautschuk, Styrol-Butadien-Kautschuk, Acrylnitril-Butadien-Kautschuk, Butylkautschuk, Silicon-Kautschuk, hydriertem Acrylnitrilbutadien-Kautschuk, Polyethylenterephthalat, Polyethylen, insbesondere hochmolekularem und/oder ultrahochmolekularem Polyethylen, und ggf. Zusätzen, beispielsweise Puder, Silikonöl, Wachs, Mikroballons, Molybdänsulfid, Graphit, Talkum, Teflonpulver und/oder Selbstschmierungs-Additiven, insbesondere Polyacetal, Polyphenylensulfid, Polyphthalamid und/oder Polyetheretherketon, und ggf. einer Beschichtung, beispielsweise mittels Teflon und/oder einer metallisierten Beschichtung, Glas und/oder Plexiglas, insbesondere Polyurethan und/oder Polymethylmethacrylat, aufweist.
